(19) Europäisches Patentamt
European Patent Office
Office européen des brevets

(11) Numéro de publication: **0 161 212 B1**

(12) # FASCICULE DE BREVET EUROPEEN

(45) Date de publication du fascicule du brevet: **24.08.88**

(51) Int. Cl.⁴: **C 08 B 37/08**, A 61 K 7/48

(21) Numéro de dépôt: **85810168.6**

(22) Date de dépôt: **16.04.85**

(54) **Polymère glycosaminoglycanique utilisable en cosmétologie.**

(30) Priorité: **17.04.84 CH 1923/84**

(43) Date de publication de la demande: **13.11.85 Bulletin 85/46**

(45) Mention de la délivrance du brevet: **24.08.88 Bulletin 88/34**

(84) Etats contractants désignés: **AT BE CH DE FR GB IT LI LU NL SE**

(56) Documents cités:
GB - A - 458 839

CHEMICAL ABSTRACTS, vol. 88, no. 2, 9 janvier 1978, page 103, no. 8869c, Columbus, Ohio, US
CHEMICAL ABSTRACTS, vol. 72, no. 12, 23 mars 1970, page 284, no. 58988f, Columbus, Ohio, US; S. CURRI et al.: "Skin-moisturizing action of a new diachysis factor"
ADVANCES IN CARBOHYDRATE CHEMISTRY AND BIOCHEMISTRY, vol. 29, 1974, pages 305-358, Academic Press, New York, US; JOHN F. KENNEDY: "Chemically reactive derivatives of polysaccharides"

(73) Titulaire: **BATTELLE MEMORIAL INSTITUTE, 7 route de Drize, CH-1227 Carouge/Genève (CH)**

(72) Inventeur: **Sachetto, Jean-Pierre, Rue des Chênes, F-74160 Saint-Julien en Genevois (FR)**
Inventeur: **Bedert, Nicole, 3, Rue Viollier, CH-1207 Genève (CH)**

(74) Mandataire: **Dousse, Blasco et al, 7, route de Drize, CH-1227 Carouge/Genève (CH)**

EP 0 161 212 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un copolymère de formule

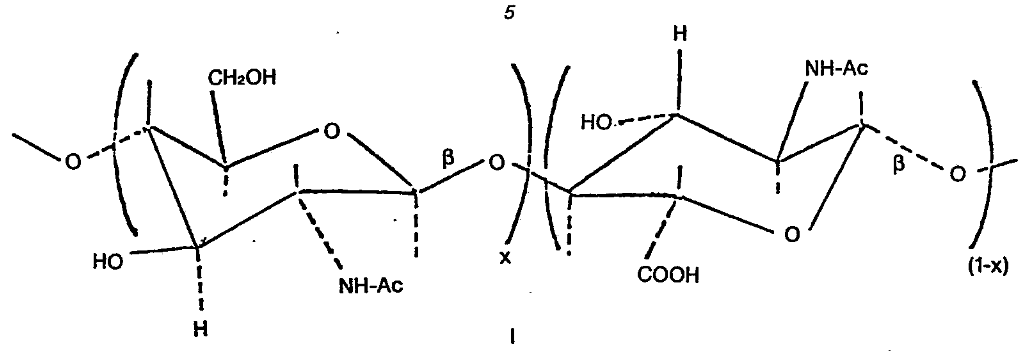

I

où x est compris entre 0,1 et 0,95 et dont le poids moléculaire est compris entre des valeurs de l'ordre de $10^4$ à $10^7$ D ainsi que ses sels alcalins, un procédé de fabrication et son utilisation en cosmétologie.

Comme on peut le voir de par la formule ci-dessus, ce copolymère est un glycosaminoglycane composé d'unités N-acétyl-D-glucosamine et d'unités acide N-acétyl-glucuronique. En général, pour 100 unités pyrannosiques, il comprend de 5 à 90 unités d'acide 2-N-acétyl-glucuronique, les unités restantes étant celles de 2-N-acétyl-glucosamine.

Le polymère est voisin, structurellement, de l'acide hyaluronique (HA), un mucopolysaccharide naturel présentant en alternance des liaisons β-1,3-glucuronique et β-1,4-glucosaminidique. Le poids moléculaire de l'HA est généralement compris entre 50 000 et 8 000 000. L'HA est présent dans de nombreux tissus animaux et, notamment, dans la peau où son rôle est important en raison de son remarquable pouvoir de rétention d'eau (voir FR-A-2,478,468 ainsi que les références cités dans ce document, à savoir: Meyer et al. J. Biol. Chem. 107, p. 629 [1934], J. Biol. Chem. 114, p. 689 [1936]; Balazs, Fed. Proc. 17, p. 1086 [1958]; Laurent et al., Biochim. Biophys. Acta 42, p. 476 [1960]; Weissman et al., J. Am. Chem. Soc. 76, p. 1753 [1954]; Meyer, Fed. Proc. 17, p. 1075 [1958]; USA-A-4,141,973).

Or, il est connu (voir D.C. STEINBERG, Relata Technica N30 [1982], p. 65–68) que la douceur et la flexibilité de la couche cornée, qui constitue la couche supérieure de la peau, dépend de la teneur en humidité des canaux intercellulaires de cette couche. Aussi les cosmétologues et les médecins-chercheurs ont-ils longuement cherché à trouver les moyens pour restaurer ces qualités vitales de la peau lorsqu'elles ont disparu, comme cela se produit au cours du processus naturel de vieillissement, et dans les cas de sécheresse intense.

La peau humaine est en effet constituée par deux couches: une couche externe appelée couche cornée (épiderme) et, au-dessus, une couche interne (derme) appelée couche de Malpighi. Cette dernière contient la première couche de cellules épithéliales vivantes. La couche cornée n'a que quelques microns d'épaisseur; autrement dit, son épaisseur est constituée par environ 10 à 20 cellules. La partie la plus externe de ces 10 à 20 cellules n'est formée que de cellules mortes. Les plus internes de ces 10 à 20 cellules, c'est-à-dire cellules qui sont les plus proches de la couche de Malpighi, constituent du tissu vivant. Les cellules comprises entre ces deux groupes sont à différents stades de mortification et, à mesure que de nouvelles cellules sont régénérées dans la couche de Malpighi, il y a une desquamation constante des cellules externes mortes. Au cours du processus naturel de vieillissement, la vitesse à laquelle les cellules âgées et mortifiées de la couche cornée sont remplacées par des cellules nouvellement produites dans la couche de Malpighi décroît et cette modification entre en ligne de compte, au moins en partie, en ce qui concerne l'aspect externe de la peau. Par ailleurs, le derme étant un réseau tridimensionnel de fibres de collagène et d'élastine noyées dans une gelée riche en acide hyaluronique, la structure intime de cette matière composite détermine la stabilité de la peau et, par voie de conséquence, son apparence extérieure. Lors du processus de vieillissement, le renouvellement en acide hyaluronique et autres substances rétentrices d'humidité étant de moins en moins assuré, la peau se dessèche, se ride et prend un aspect parcheminé contre lequel les cosmétologues se sont éfforcés de lutter, notamment par l'utilisation de crèmes hydratantes à base d'acide hyaluronique ou de ses sels solubles dans l'eau.

On trouvera des informations détaillées sur l'utilisation de l'HA en cosmétologie dans la référence suivante: Mucopolysaccharides for cosmetics, D.C. STEINBERG, Relata Technica 30 (1982), pages 65–68.

Par ailleurs, on peut encore citer, dans ce domaine général, les références suivantes:

Le document GB-A-458,839 (DU PONT) devulgue un procédé pour desacétyler la chitine en chitosanine ainsi que l'oxydation de la chitine par le permanganate, l'hypochlorite et autres substances similaires. Le produit de la présente invention ne figure cependant pas dans ce document.

Le document Riv. Ital. Essenze, Profumi, Piante Offic., Aromi, Saponi, Cosmet., Aerosol 1969, 51(8), 416–28 (Chemical Abstract 72 58988) décrit un produit hydratant de la peau dont la formule structurale présente quelque analogie avec celle du produit de

l'invention. Il s'agit là cependant d'un poly-acylhexosaminoglycane et non d'un polyglycoside comme l'acide hyaluronique.

Le document JP-A-81/158,706 (Chemical Abstracts 96 129594) décrit l'utilisatiion pour protéger la peau du dessèchement de mucopolysaccharides extraits de tissus cartilagineux de certains animaux. Parmi ces composés sont cités l'acide hyaluronique et les sulfates de chondroïtine et de mucoïtine. Le produit de l'invention n'est cependant pas cité.

Le document «The Carbohydrates», éditeurs W. PIGMAN et D. HORTON, Academic Press (1980), page 1142 cite l'action de l'acide chromique sur le chitosane comme fournissant un aminodesoxyglycopyrannuranane et non pas le produit de la présente invention.

L'acide hyaluronique et ses dérivés étant difficiles à préparer et très onéreux, on a cherché à le remplacer par un produit de propriétés similaires meilleur marché. Le polymère de l'invention convient à ce remplacement car ses propriétés de solubilité, de rétention d'eau (ainsi que celles de ses sels) sont voisines de celles de l'HA et de l'HANa.

Suivant un procédé de préparation du polymère de l'invention, on soumet de la chitine à un processus d'oxydation suivant la réaction:

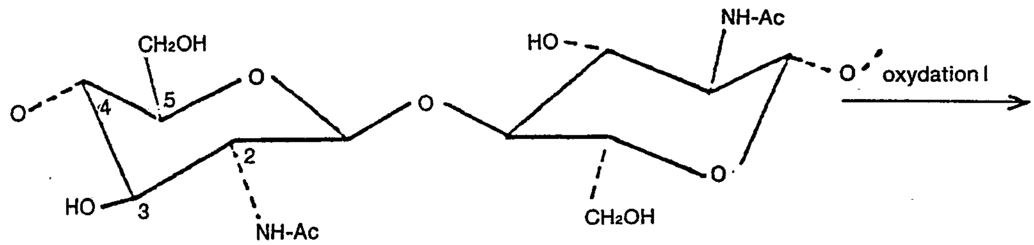


I

de manière à convertir une proportion convenable des groupes 5- ou 5'-hydroxyméthyl en groupes carboxyliques, cette proportion variant entre 5 et 90%.

Comme oxydant, on utilise le $NO_2$ gazeux ou son dimère liquide ($N_2O_4$), éventuellement $CrO_3$. De préférence, on utilise de l'oxyde d'azote liquide ou gazeux dans lequel on disperse la chitine par agitation, celle-ci restant en contact avec l'oxydant d'une heure à 5 jours à une température de l'ordre de 10 à 20 °C à pression atmosphérique. Ces limites ne sont cependant pas critiques et on peut les dépasser, notamment si on travaille sous pression afin de pouvoir opérer à des températures supérieures à 20 °C (température d'ébullition du $N_2O_4$ sous pression atmosphérique = 21 °C). Suivant les conditions le taux d'oxydation des groupes $CH_2OH$, de la chitine en groupes COOH peut être de 5 à 90%.

Lorsque la réaction est terminée, on sépare le polymère de l'excès d'oxyde d'azote, par exemple par essorage. Le produit brut consiste en une poudre de couleur légèrement jaunâtre, partiellement soluble dans l'eau et composée d'un mélange de molécules de poids moléculaire variant entre de larges limites. Le polymère peut être fractionné par reprécipitation fractionnée de ses solutions dans le méthanol ou l'éthanol au moyen de solvants tels que le propanol, l'isopropanol, le butanol et autres alcools en $C_4$ à $C_6$, l'acétone, le dioxanne, le THF, le monoglyme et autres solvants hydro- et alcoolocompatibles ou, par dialyse, au moyen de membranes d'ultrafiltration de perméabilités variées en utilisant des solutions du produit I dans les solvants précités ou des mélanges d'eau et de ceux-ci. On peut décolorer le polymère brut par réduction, par exemple au moyen de $NaBH_4$ en solution dans un alcool, par exemple EtOH.

Ainsi, suivant un procédé de préparation avantageux, on met en agitation, 24 h à 20 °C environ, 10 à 20 g de chitine dans 50–100 g de $N_2O_4$ liquide, le récipient laboratoire étant mini d'un refrigérant à reflux permettant de condenser les vapeurs de l'oxyde d'azote se formant au dessus du mélange réactionnel; puis on décante le liquide à froid (celui-ci peut être d'ailleurs réutilisé sans autre pour une nouvelle préparation) et lave le solide formé par un solvant chloré, par exemple $CCl_4$ ou le chloroforme. On recueille alors le produit I brut sous forme d'une poudre jaunâtre, le rendement étant voisin de 100%. On détermine le taux d'oxydation (c'est-à-dire le taux de conversion des groupes $CH_2CH$ en groupes carboxyliques) par ébullition d'un échantillon dans du HCl aqueux à 10 à 20%, entraînement du $CO_2$ formé par décarboxylation par un courant de gaz inerte (par exemple $N_2$) et dosage de ce $CO_2$ par transformation en $BaCO_3$ suivant les moyens habituels. On a ainsi pu déterminer les valeurs de x dans la formule I et on cite, notamment, les chiffres suivants: 0,132; 0,2588; 0,348; 0,573; 0,6725.

On purifie de préférance le produit brut en le dissolvant dans un souvant tel que le méthanol ou l'éthanol et en le reprécipitant par un alcool de poids moléculaire supérieur ou un autre solvant compatible tel que les solvants précités. L'isopropanol convient bien pour cela, le produit précipité purifié étant alors pratiquement incolore (poudre blanche) et ayant un degré d'oxydation quelque peu supérieur à celui du produit brut.

On peut également purifier le produit c'est-à-dire le séparer en une fraction aisément hydrosoluble d'une fraction moins aisément hydrosoluble (parce que moins oxydée) par traitement à l'eau suivi d'une filtration ou centrifugation de la suspension ainsi obtenue.

On peut également fractionner le produit de l'invention en le soumettant à une ultrafiltration au moyen de membranes de porosités calibrées, cette technique permettant ainsi d'isoler une série de fractions de poids moléculaire compris dans une certaine gamme, par exemple entre 20 000 et 50 000 ou entre 50 000 et 200 000.

On peut utiliser le produit I en cosmétologie, par

exemple pour la préparation de produits hydratants tels que crèmes et lotions de beauté et autres. Pour ce faire, on mélange avec les ingrédients habituels de ces cosmétiques le produit I, de préférence à l'état purifié incolore, sous forme de gels aqueux obtenus par additon d'eau à l'acide, soit libre, soit au moins partiellememt salifié par un métal alcalin. Pour réaliser une telle salification, on traite le polymère I, dans sa forme acide, par une base alcaline, par exemple le carbonate ou le bicarbonate de sodium. Lorsque le produit cosmétique est une émulsion huile/eau ou eau/huile, on peut ajouter le produit I à sec, sa transformation en gel hydraté se produisant ultérieurement. On peut ajouter, en poids, d'environ 0,1 à 10% de la composition.

Il est à noter que les fractions particulièrement avantageuses du produit de l'invention sont celles, hydrosolubles, de poids moléculaire moyen compris entre 50 000 et 500 000 et dont le degré d'oxydation est supérieur à 50%.

Les exemples qui suivent illustrent l'invention pour la compréhension desquels on se réfèrera au dessin annexé.

La fig. 1 est un diagramme représentant l'absorption d'eau (en % de poids) du produit de l'invention en fonction de l'humidité de l'atmosphère dans laquelle il est placé à température ambiante.

La fig. 2 est un diagramme de la perte d'eau (en % de poids) du produit de l'invention lorsque celui-ci, après avoir été hydraté, est maintenu en atmosphère sèche à température ambiante.

Exemple 1

On a placé 16,4 g de chitine moulue dans un ballon muni d'un réfrigérant à reflux et d'un agitateur et on a ajouté 170 ml de $NO_2$ liquide (l'adjonction s'est faite à froid pour éviter une évaporation prématurée de l'oxyde d'azote). On a mis le tout 24 h en agitation à 20 °C, après quoi on a décanté avec précaution le liquide (celui-ci peut être réutilisé sans autre pour une oxydation ultérieure) et on a lavé le solide avec du $CCl_4$. Le rendement était pratiquement quantitatif.

On a repris le solide dans environ 50 ml de méthanol où une partie s'est dissoute. On a filtré la fraction insoluble (A) (rendement 47,6%) et on a évaporé la solution méthanolique ce qui a fourni une seconde fraction (D) (43,8%). Par analyse des groupes COOH, on a constaté que la fraction A en comprenait 6,64%, ce qui correspond à un taux d'oxydation de 32,75% (taux de transformation des groupes $-CH_2OH$ en groupes $-COOH$, 100% désignant une transformation complète). La fraction D a donné les résultats suivants: % de COOH = 15,03; taux d'oxydation: 74,12%. Les valeurs de x dans la formule I étaient donc les suivantes: A, x = 0,6725 et D, x = 0,2588.

Exemple 2

On a mis en communication par un tube de fort diamètre la partie supérieure d'un ballon muni d'un réfrigérant à reflux et contenant 20,5 g de chitine moulue avec celle d'un second ballon contenant 15 ml de $NO_2$ (l'oxyde d'azote ayant été introduit dans le second ballon puis congelé), le tout de manière que les vapeurs de $NO_2$ se dégageant progressivement par réchauffage de ce $NO_2$ pénètrent dans le premier ballon et imprègnent la chitine placée dans celui-ci.

Après 4 jours à température ambiante, on a repris le solide par du $CCl_4$ puis par 150 ml de méthanol ce qui en a dissous une partie. Le rendement en chitine oxydée (B) insoluble dans le méthanol était de 8,6 g; % de COOH = 8,53; taux d'oxydation 42,7%, poudre blanche.

Exemple 3

On a procédé comme décrit à l'Exemple 1 en utilisant 10 g de chitine moulue et 100 ml de $NO_2$ liquide, mélange qu'on a maintenu 24 h à 19–19,5 °C. Après décantation du liquide, on a lavé le solide au chloroforme et on l'a séché, ce qui a fourni 11,31 g de produit oxydé (E). Analyse: % de COOH = 17,6; taux d'oxydation 86,8%.

On a prélevé 2 g du produit (E) et on a agité cet échantillon 1 heure à température ambiante dans 100 ml d'éthanol absolu avec 2 g de $NaBH_4$. On a alors détruit l'excédent de borohydrure avec de l'acide acétique pur, puis on a récolté le solide par filtration et on l'a séché. Echantillon (C), poudre blanche. Analyse: teneur en COOH = 13,22%; taux d'oxydation = 65,2%.

Exemple 4

On a transformé des aliquots des échantillons A à E des Exemples précédents en sels de sodium corespondants de la manière suivante: on a mis en suspension dans de l'eau environ 2–3 g de solide et on a neutralisé le pH du milieu par addition de bicarbonate de soude. Puis on a filtré le solide et on l'a séché à l'air sec au dessicateur.

On a placé des échantillons pesés exactement des sels de sodium A à E secs (ainsi qu'un échantillon de contrôle constitué par de la chitine non oxydée Y) dans des conditions d'humidité contrôlée constante (enceintes fermées contenant de l'air et de la vapeur d'eau) et on a mesuré à intervalles la quantité d'eau absorbée par la poudre. Le graphique de la fig. 1 montre cette variation de poids en fonction du temps et indique que la capacité totale d'absorption augmente en fonction du taux d'oxydation.

On a ensuite placé les échantillons humidifiés en atmosphère contrôlée sèche (18,8% d'humidité relative à température ordinaire) et on a mesuré le taux de séchage (désorption d'humidité). Les résultats figurent à la fig. 2 où on a également reporté, courbe désignée par X, les valeurs correspondant à un échantillon témoin de pyrrolidone carboxylate de sodium, un agent hydratant à faible poids moléculaire également utilisé en cosmétique (voir International Journal of Cosmetic Sciences 3 [1981], 101–113).

Exemple 5

A 65 °C on a mélangé sous agitation les ingrédients suivants (% en poids):

| | |
|---|---|
| Polyethylène glycol (PEG-8) | 9 |
| Ticlycéride d'acides gras $C_{10}$–$C_{18}$ | 18 |
| Tristéarine | 8 |
| Phenoxyethoxyparabène (fongicide) | 1,5 |

| | |
|---|---|
| Non saponifiable de l'huile de soya | 0,02 |
| Hydrorhamnosane | 1,0 |
| Propylène glycol | 6 |
| Stéarate d'isocét-10-yle | 1,2 |

puis, à 40 °C, on a ajouté ce mélange à 45 g d'eau distillée. On a alors encore ajouté, dans l'ordre, avec agitation, 2% de collagène hydrosoluble, 0,04% d'essence de rose synthétique, 0,3 g d'oxyde de chitine (échantillon C préparé à l'Exemple 3), puis 6,14% d'eau, toujours à 40 °C.

On a utilisé l'émulsion homogène ainsi obtenue comme crème hydratante pour la peau avec des résultats comparables à celle d'une crème témoin de même composition, mais contenant comme agent hydratant, du hyaluronate de sodium HNa.

Exemple 6

On a mis en suspension 1 kg de chitine commerciale dans 4 l de solution aqueuse de NaOH 11N rafroidie; après environ 12–24 heures au froid, on a dilué à l'eau, filtré le solide et on l'a lavé à l'eau jusqu'à disparition de l'alcali; puis on l'a séché. Ce traitement améliore la dispersibilité de la chitine dans la réaction d'oxydation qui suit:

On a mis en agitation lente 7 heures à température ambiante 100 g de chitine prétraitée comme décrit ci-dessus dans 1 l de $N_2O_4$ liquide, puis on a décanté le liquide, lavé le solide au $CHCl_3$ et on l'a séché au rotavapor.

On a ensuite repris le produit à l'eau, ajusté le pH à 4 avec de la soude caustique diluée et soumis le tout à une dialyse dans l'eau courante, une nuit à température ambiante et en utilisant une membrane permettant d'éliminer les produits de poids moléculaire (MW) inférieur à 1000.

On a alors récolté 45,5 g de produit titrant 37% d'oxydation (taux des COOH = 7,48%).

On a repris ce produit dans 2,5 l d'eau dont le pH a été amené à 6,9–7,1 par une solution concentrée (50 g/l) d'hydrogénocarbonate de sodium. On a procédé à une centrifugation ce qui a permis d'isoler un culot (B) de 18,3 g (après séchage) et une solution transparente qui, après évaporation sous pression réduite, as fourni 27,2 g d'une poudre incolore (A). Les résultats analytiques des fractions A et B étaient les suivants: taux de COOH: A 12,32; B 2,97; pourcent d'oxydation: A 61%; B 15%.

On a soumis le produit A à une ultracentrifugation, membrane MW 50 000. On a ainsi recueilli 15,3 g de produits MW<50 000 et 11,6 g de produit MW<50 000. Cette dernière fraction s'est révélée particulièrement avantageuse comme agent réhydratant en cosmétologie.

On notera que pour préparer le produit suivant l'invention, on peut également travailler en milieu acide phosphorique 80% en présence de nitrite de sodium comme oxydant (formation de $NO_2$ par l'action de l'acide sur le nitrite alcalin).

**Revendications**

**pour les Etats contractants: BE, CH, DE, FR, GB, IT, LU, NL, SE**

1. Copolymère de formule

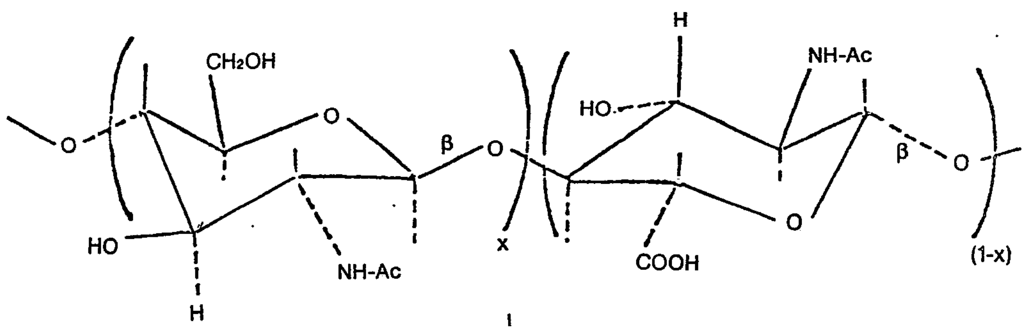

I

où x est compris entre 0,1 et 0,95 et dont le poids moléculaire est de $10^4$ à $10^7$ D ainsi que ses sels de métaux alcalins.

2. Copolymère suivant la revendication 1 caractérisé par le fait que x a les valeurs suivantes: 0,132; 0,2588; 0,348; 0,573 et 0,6725.

3. Utilisation du composé I suivant la revendication 1 en cosmétologie en tant qu'ingrédient hydratant de la peau.

4. Utilisation suivant la revendication 3, caractérisé par le fait qu'on incorpore le composé I dans une composition de crème ou de lotion hydratante au taux de 0,1 à 10% en poids de la composition.

5. Procédé de préparation du composé I, caractérisé par le fait qu'on soumet de la chitine à une oxydation par l'oxyde d'azote liquide ou gazeux, le taux d'oxydation du groupe $-CH_2OH$ de la chitine en groupe $-COOH$ du composé I étant de 5 à 90%.

6. Procédé suivant la revendication 5, caractérisé par le fait qu'on purifie le composé I obtenu par oxydation, par précipitation fractionnée, en le dissolvant dans du méthanol et en le reprécipitant par un alcool de poids moléculaire plus élevé.

7. Procédé suivant la revendication 5, caractérisé par le fait qu'on purifie le composé I par dialyse dans l'eau au moyen d'une membrane semi-perméable.

## Revendications

**pour l'Etat contractant: AT**

1. Procédé de préparation du copolymère de formule

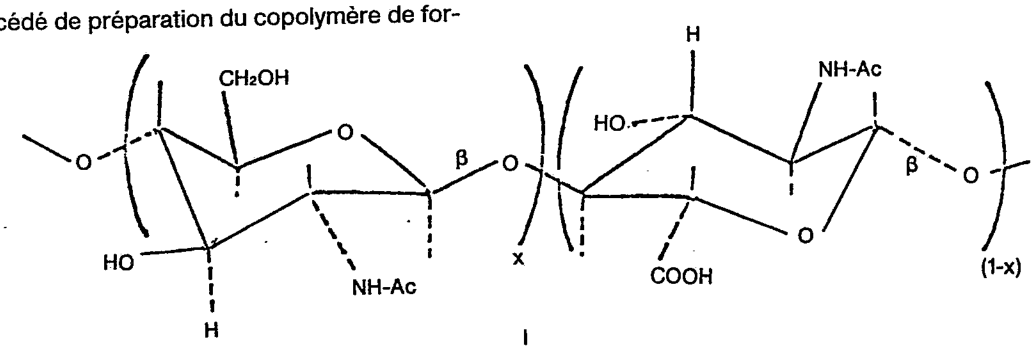

I

utilisable en cosmétique, où x est compris entre 0,1 et 0,95 et dont le poids moléculaire est de $10^4$ à $10^7$ D ainsi que ses sels de métaux alcalins, caractérisé par le fait qu'on soumet de la chitine à une oxydation par l'oxyde d'azote liquide ou gazeux, le taux d'oxydation du groupe $-CH_2OH$ de la chitine en groupe $-COOH$ du composé I étant de 5 à 90%.

2. Procédé suivant la revendication 1, caractérisé par le fait qu'on purifie le composé I obtenu par oxydation, par précipitation fractionnée, en le dissolvant dans du méthanol et en le reprécipitant par un alcool de poids moléculaire plus élevé.

3. Procédé suivant la revendication 1, caractérisé par le fait qu'on purifie le composé I par dialyse dans l'eau au moyen d'une membrane semi-perméable.

4. Procédé suivant la revendication 1, caractérisé par le fait qu'on choisit le temps de réaction entre environ 4 à 24 heures de manière que, suivant la durée, x ait les valeurs suivantes: 0,132; 0,2588; 0,348; 0,573 et 0,6725.

5. Procédé suivant la revendication 1, caractérisé par le fait que, pour l'obtention des sels alcalins, on traite le composé I dans lequel les groupes . carboxyliques sont acides par un sel ou un hydroxyde alcalin.

6. Utilisation du composé I en cosmétologie en tant qu'ingrédient hydratant de la peau.

7. Utilisation suivant la revendication 6, caractérisé par le fait qu'on incorpore le composé I dans une composition de crème ou de lotion hydratante au taux de 0,1 à 10% en poids de la composition.

## Patentansprüche

**für die Vertragsstaaten BE, CH, D, F, GB, I, LU, NL, SE**

1. Copolymer der Formel

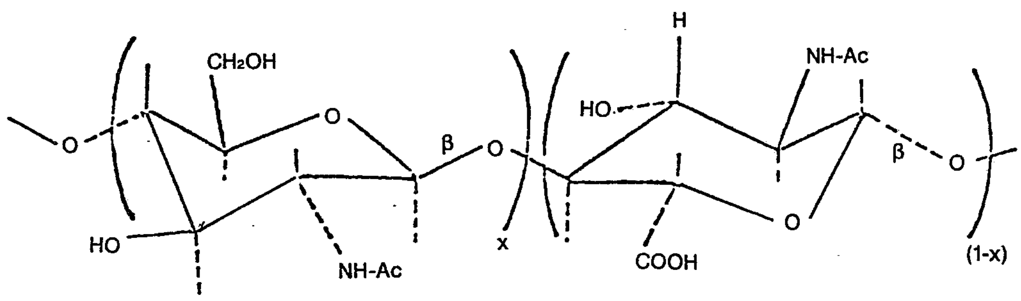

I

wo x zwischen 0,1 und 0,95 liegt und dessen Molekulargewicht $10^4$ bis $10^7$D ist, sowie dessen alkalische Salze.

2. Copolymer nach Anspruch 1, dadurch gekennzeichnet, daß x folgende Werte hat: 0,132; 0,2588; 0,348; 0,573 und 0,6725.

3. Verwendung der Verbindung I nach Anspruch 1, in der Schönheitskunde als hydrierender Bestandteil der Haut.

4. Verwendung nach Anspruch 3, dadurch gekennzeichnet, daß man die Verbindung I in einer Cremezusammensetzung oder einer Feuchtigkeitslotion in einem Ausmaß von 0,1 bis 10 Gew.% der Zusammensetzung einbringt.

5. Verfahren zur Herstellung der Verbindung I dadurch gekennzeichnet, daß man Chitin einer Oxydation mit flüssigem oder gasförmigem Stickoxyd unterwirft, wobei das Oxydationsausmaß der $-CH_2OH$-Gruppe des Chitins in die $-COOH$-Gruppe der Verbindung I 5 bis 90% ist.

6. Verfahren nach Anspruch 5, dadurch gekennzeichnet, daß man die durch Oxydation erhaltene Verbindung I durch fraktionierte Fällung reinigt, indem man sie in Methanol löst und sie mit einem Alkohol erhöhten Molekulargewichtes wieder ausfällt.

7. Verfahren nach Anspruch 5, dadurch gekennzeichnet, daß man die Verbindung I durch Dialyse in Wasser mittels einer halbdurchlässigen Membran reinigt.

**Patentansprüche**

**für den Vertragsstaat AT**

1. Verfahren zur Herstellung des Copolymers der Formel

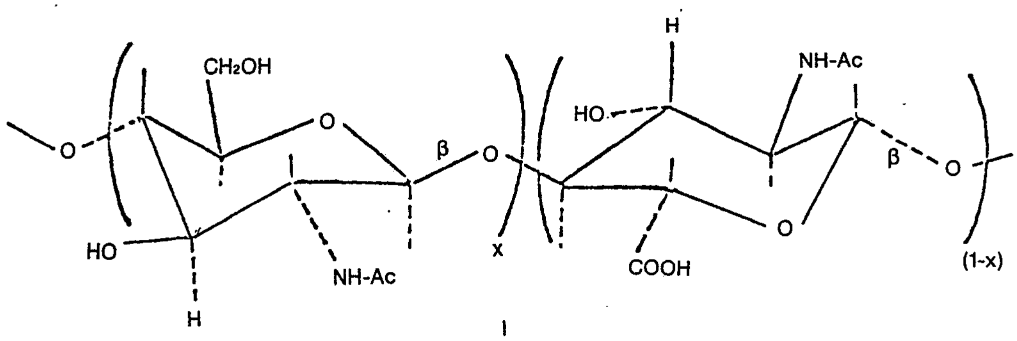

I

das in der Kosmetik verwendbar ist, wobei x zwischen 0,1 und 0,95 liegt und dessen Molekulargewicht $10^4$ bis $10^7$ D ist, sowie dessen alkalische Salze, dadurch gekenzeichnet, daß man Chitin einer Oxydation mit flüssigem oder gasförmigem Stickoxid unterwirft, wobei das Oxydationsausmaß der $-CH_2OH$-Gruppe der Chitins in die $-COOH$-Gruppe der Verbindung I 5 bis 90% ist.

2. Verfahren nach Anspruch 1, dadurch gekennzeichnet, daß man die durch Oxydation erhaltene Verbindung I durch fraktionierte Fällung reinigt, indem man sie in Methanol löst und sie mit einem Alkohol erhöhten Molekulargewichtes wieder ausfällt.

3. Verfahren nach Anspruch 1, dadurch gekennzeichnet, daß man die Verbindung I durch Dialyse in Wasser mittels einer halbdurchlässigen Membran reinigt.

4. Verfahren nach Anspruch 1, dadurch gekennzeichnet, daß man die Reaktionszeit zwischen 4 und 24 Stunden wählt, derart daß x während der Dauer folgende Werte hat: 0,132; 0,2588; 0,348; 0, 573 und 0,6725.

5. Verfahren nach Anspruch 1, dadurch gekennzeichnet, daß zur Herstellung der alkalischen Salze man die Verbindung I, in der die Carboxylgruppen Säuren sind, mit einem Salz oder einem alkalischen Hydroxid behandelt.

6. Verwendung der Verbindung I in der Schönheitskunde als hydrierender Bestandteil der Haut.

7. Verwendung nach Anspruch 6, dadurch gekennzeichnet, daß man die Verbindung I in einer Cremezusammensetzung oder einer Feuchtigkeitslotion in einem Ausmaß von 0,1 bis 10 Gew.% der Zusammensetzung einbringt.

**Claims**

**for the contracting states BE, CH, D, F, GB, I, LU, NL, SE**

1. A co-polymer with the formula

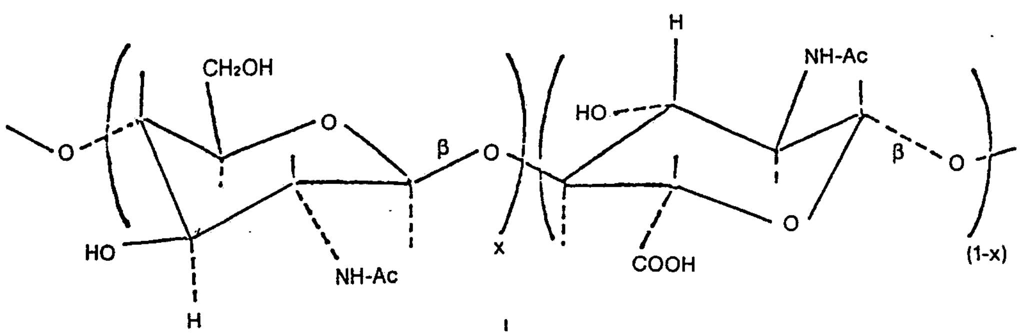

I

where x is between 0.1 and 0.95 and the molecular weight of which is between $10^4$ and $10^7$ D and its alkaline metal salts.

2. A copolymer according to claim 1, characterized in that x has the following values: 0.132; 0.2588; 0.348; 0.573 and 0.6725.

3. Use of compound I according to claim 1 in cosmetology as a skin moisturizing ingredient.

4. Use according to claim 3, characterized in that the compound I is incorporated in a composition of moisturizing cream or lotion at a level of 0.1 to 10% by weight of the composition.

5. A process for the preparation of compound I, characterized in that chitin is subjected to oxidation by liquid or gaseous nitrogen oxide, the extent of oxidation for the $-CH_2OH$ group of the chitin into $-COOH$ group of the compound I being 5 to 90%.

6. Process according to claim 5, characterized in that the compound I obtained by oxidation is purified by fractionated precipitation by dissolving it in methanol and reprecipitation using an alcohol with higher molecular weight.

7. Process according to claim 1, characterized in that the compound I is purified by dialysis in water via a semi-permeable membrane.

**Claims**

**for the contracting state AT**

1. A method for preparing a co-polymer with the formula

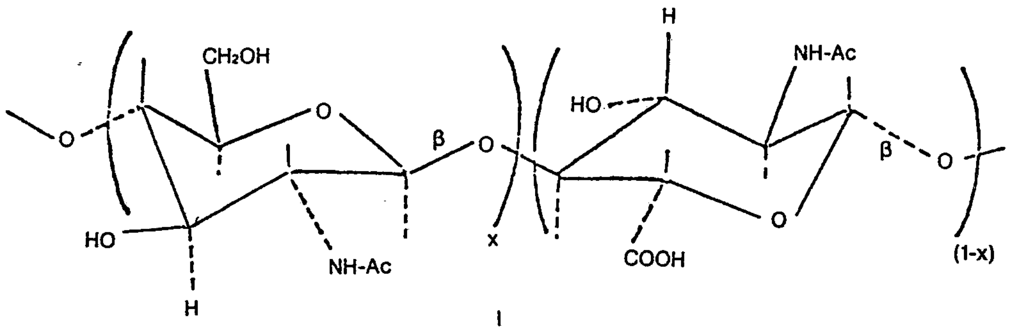

I

to be used in cosmetology where x is between 0.1 and 0.95 and the molecular wight of which is between $10^4$ and $10^7$ D and its alkaline metal salts, characterized in subjecting chitin to oxidation by liquid or gaseous nitrogen oxide, the degree of oxidation for the $-CH_2OH$ of chitin into $-COOH$ groups of compound I being 5 to 90%.

2. The method according to claim 1, characterized in that compound I obtained by oxidation is purified by fractional precipitation, by dissolving into methanol and reprecipitating with a higher molecular weight alcohol.

3. The method according to claim 1, characterized in that compound I is purified by dialyzing in water via asemi-permeable membrane.

4. A method according to claim 1, characterized in selecting the reaction time to be between about 4 and 24 hours, so that, depending on duration, x has the following values: 0.132; 0.2588; 0.348; 0.573 and 0.6725.

5. A method according to claim 1, characterized in obtaining alkali salts of compound I in which the carboxyl groups are acidic by subjecting it to an alkali salt or hydroxide.

6. The use of compound I in cosmetology as a skin moisturizing ingredient.

7. The use according to claim 6, characterized in that the compound I is incorporated in a composition of moisturizing cream or lotion at a level of 0.1 to10% by weight of the composition.

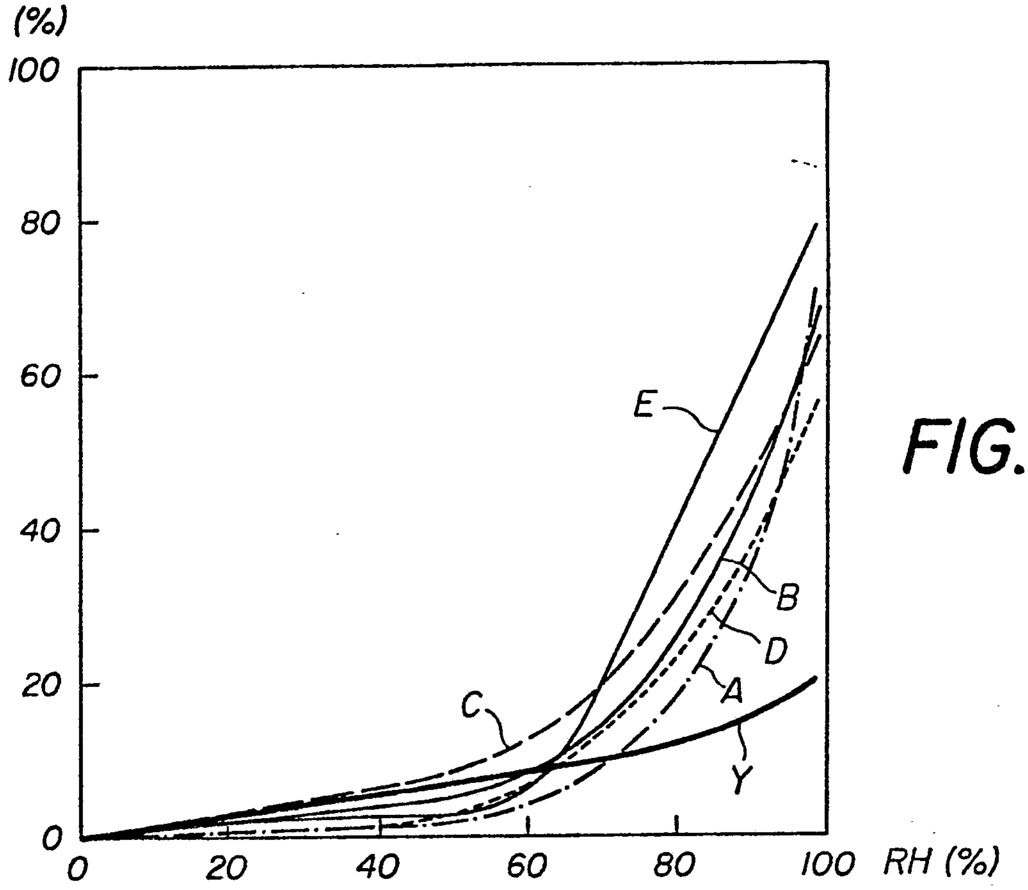
(%)
100
80
60
40
20
0
0
20
40
60
80
100
RH (%)
E
B
D
A
C
Y

FIG. 1

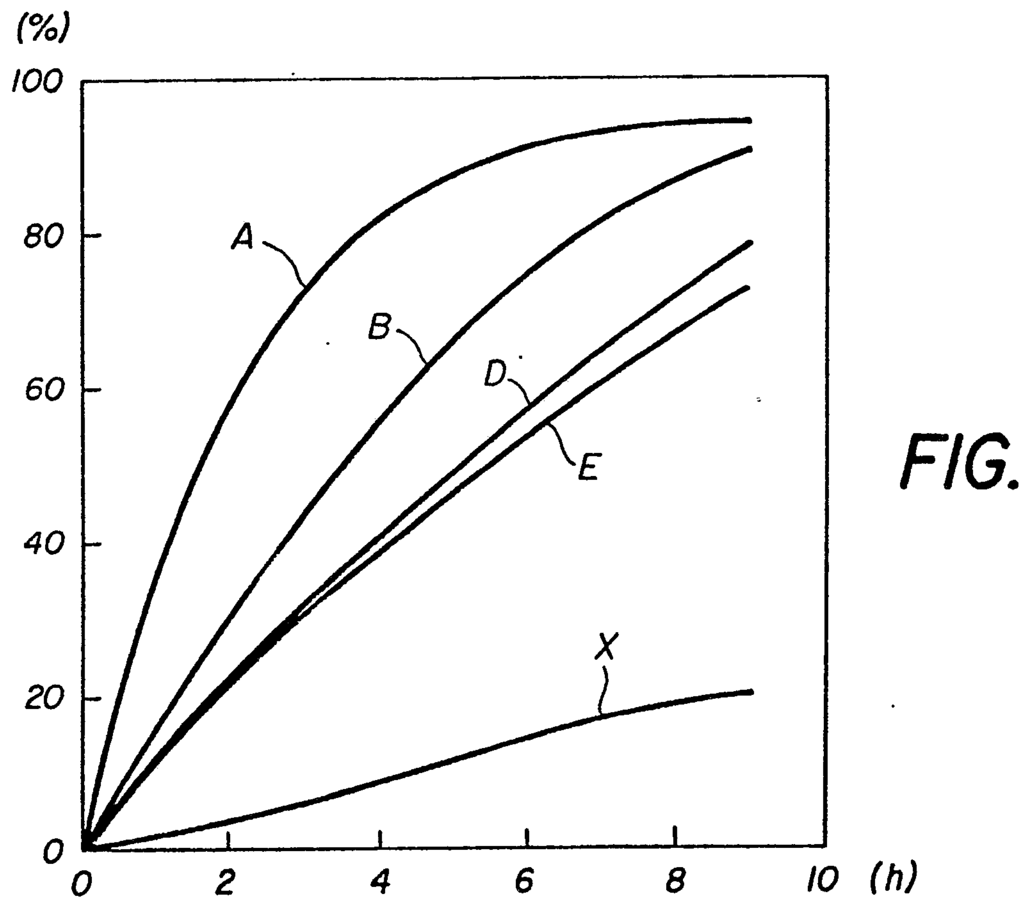
(%)
100
80
60
40
20
0
0
2
4
6
8
10
(h)
A
B
D
E
X

FIG. 2